# EUROPEAN PATENT APPLICATION

(11) **EP 2 019 097 A1**
(43) Date of publication of application: **28.01.2009**
(21) Application number: 08011984.5
(22) Date of filing: 03.07.2008
(51) Int. Cl.: C07D 295/135

(54) **Process for preparing (alphaS)-alpha-(2-methylpropyl)-2-(1-piperidinyl)benzenemethanamine**

(30) Priority: 25.07.2007 IN MA16072007
(71) Applicant: Aurobindo Pharma Limited, Hyderabad 500 038 (IN)
(72) Inventor: Mallikarjuna, Reddy Karuru, Ameerpet Hyderabad 500 038 (IN); Jayati, Mitra, Ameerpet Hyderabad 500 038 (IN); Sivakumaran, Meenakshisunderam, Ameerpet Hyderabad 500 038 (IN); Ramesh, Dandala, Ameerpet Hyderabad 500 038 (IN)
(74) Representative: Wittkopp, Alexander

(57) **Abstract**

The present invention relates to a process for the preparation of (αS)-α-(2-methylpropyl)-2- (1-piperidinyl)benzenemethanamine of Formula I, 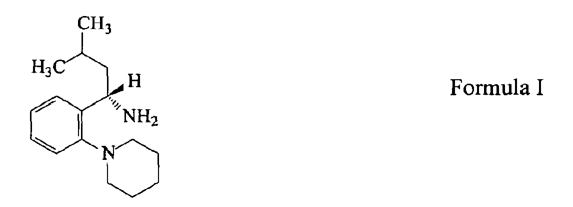 which is a key intermediate for the synthesis of Repaglinide.

## Description

### FIELD OF THE INVENTION

The present invention relates to an improved process for the preparation of (αS)-α-(2-methylpropyl)-2-(1-piperidinyl)benzenemethanamine of Formula I, 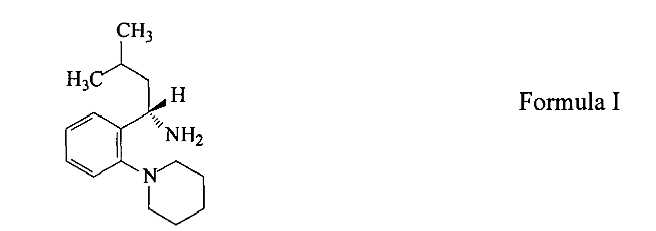 which is a key intermediate for the synthesis of repaglinide of Formula II,

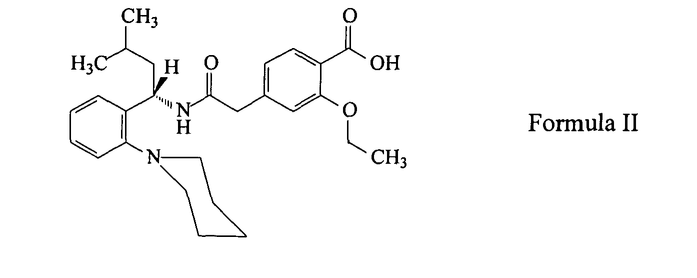

### BACKGROUND OF THE INVENTION

Repaglinide is chemically known as 2-ethoxy-4-[2-[[(1*S*)-2-methyl-1-[2-(1-piperidinyl)-phenyl]butyl]amino]-2-oxoethyl]benzoic acid. Repaglinide is marketed under the trade name PRANDIN. Repaglinide is a hypoglycemic agent useful for the treatment of type II non-insulin dependent diabetes mellitus.

In prior art Repaglinide has been synthesized via an intermediate of Formula I. The racemic amine has been resolved using N-acetyl-L-glutamic acid in US 6,143,769 and EP 0 589 874 B1. The process is as summarized below:

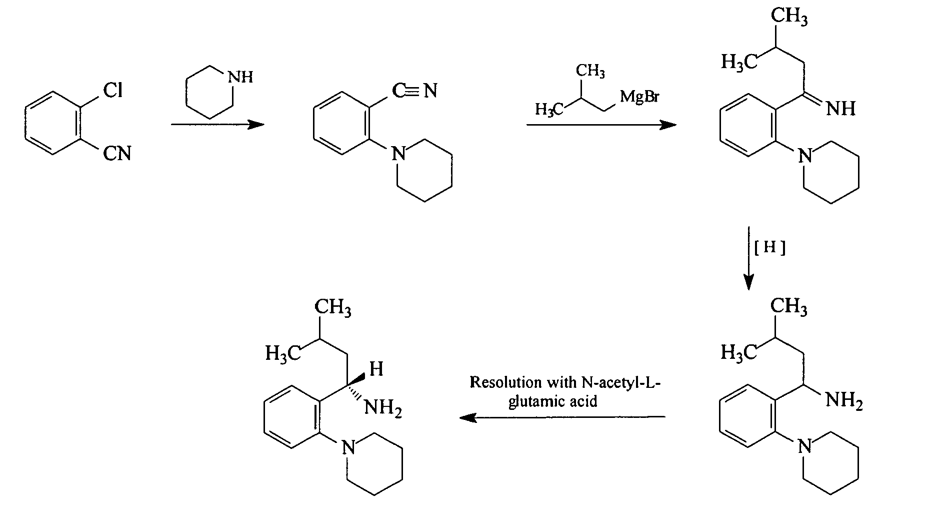

Journal of Medicinal Chemistry 1998, 41, 5219 discloses different methods to prepare chiral amines, which are as shown below:

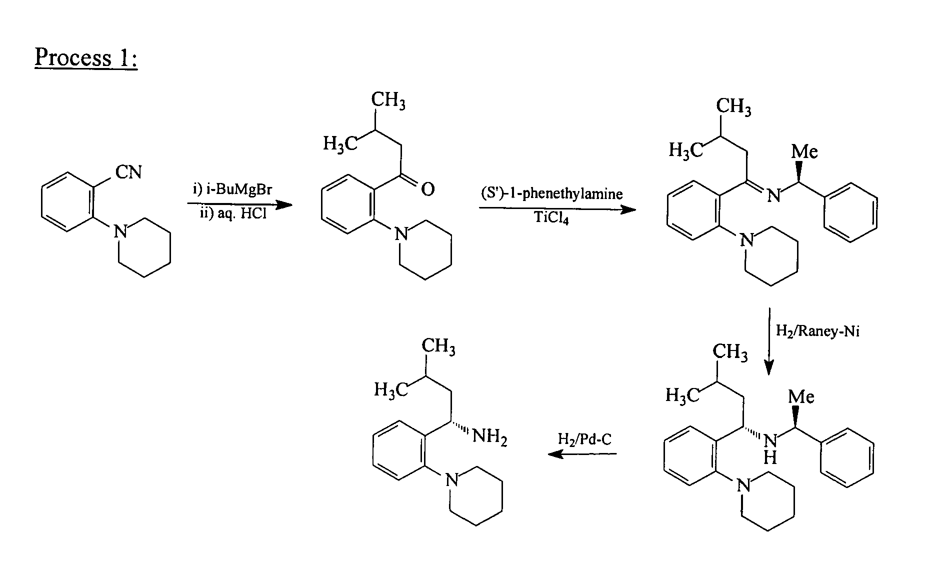

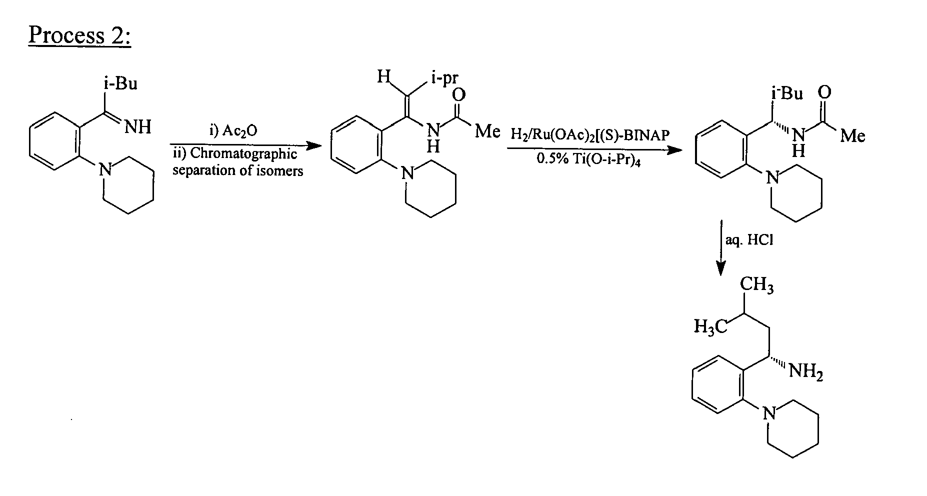

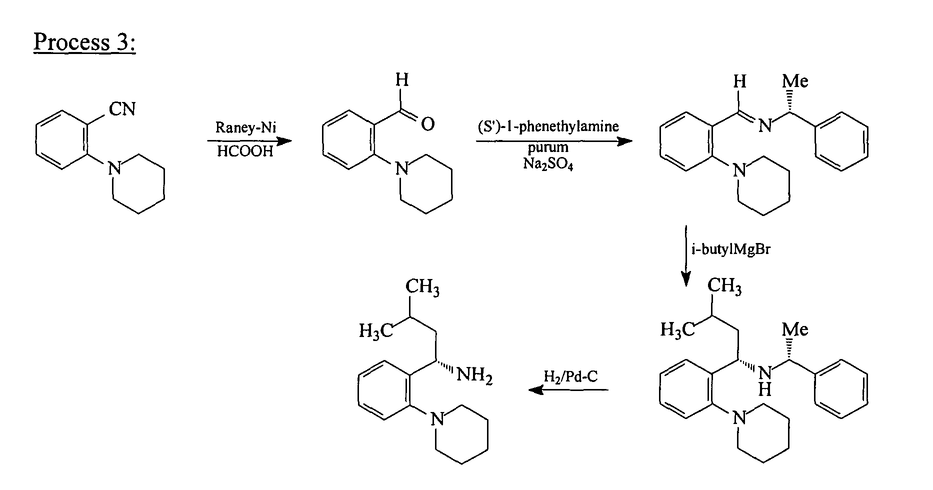

US 2007/0123564 A1 discloses a process to prepare repaglinide by resoluting the amine using a chiral reagent. The process is as shown below: 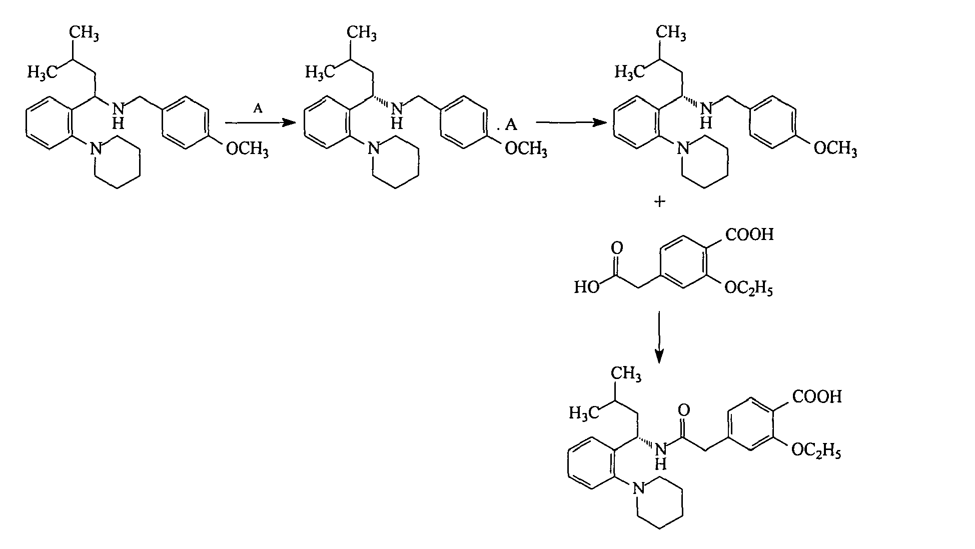 wherein A represents a chiral reagent selected from optically active carboxylic acids, e.g. mandelic acid, which is optionally substituted at the phenyl ring, optically active camphorsulphonic acid, optically active tartaric acid and optically active substituted tartaric acid or also optically active phosphoric acid, e.g. 1,1'-binaphthalin-2,2'-diyl-phosphoric acid.

The inventors have developed a more convenient process in comparison to the methods described above, where the amine has to be reacted with a substituted benzyl group and finally deprotected, which is cumbersome on a larger scale.

### OBJECTIVE OF THE INVENTION

The objective of the present invention is to provide a process for preparing pure amine, which is commercially feasible with high yield and acceptable purity suitable for making repaglinide.

Another objective of the present invention is that the process results in higher yields compared to the methods already disclosed in prior art for the manufacture of repaglinide.

### SUMMARY OF THE INVENTION

The present invention relates to an improved process for the preparation of (αS)-α-(2-methylpropyl)-2- (1-piperidinyl)benzenemethanamine of Formula I 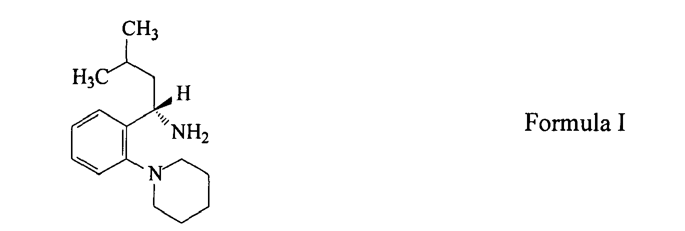 which comprises,
a) dissolving racemic amine of Formula III 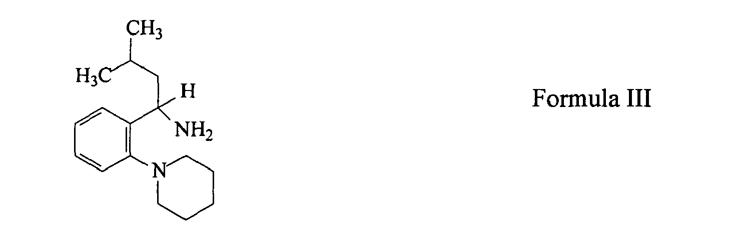 in a solvent or a mixture of solvents;
b) adding an (4*R*)-1-benzoyl-4-hydroxy-L-proline; and
c) crystallizing the diastereomeric salt and isolating the amine of Formula I.

The present invention also relates to further conversion of the amine of Formula I to repaglinide of Formula II.

### DETAILED DESCRIPTION OF THE INVENTION

The present invention relates to a process for the preparation of (αS)-α-(2-methylpropyl)-2-(1-piperidinyl)benzenemethanamine of Formula I, which comprises dissolving racemic amine in a solvent or a mixture of solvents at temperatures ranging from 0°C to 100°C, preferably at ambient temperature 25-30°C. The solvents are selected from aliphatic esters such as ethyl acetate, ketones such as acetone, alcohols such as methanol, and ethers such as t-butyl methyl ether, preferably acetone. To the above solution an (4*R*)-1-benzoyl-4-hydroxy-L-proline is added and heated to 50-65°C, preferably at 40-45°C to get a clear solution. Thereafter the solution is cooled to allow the fractional crystallization of the diastereomeric salt at a suitable rate from 30 min to 24 h, optionally in the presence of seed and isolating the crystallized product. Further crystallizing the isolated diastereomeric salt if required in a suitable solvent or mixture of solvents by dissolution followed by reprecipitation for increasing the enantiomeric purity, preferably in acetone or methanol / ethyl acetate mixture. Suspending the diastereomeric salt in aqueous medium followed by basification and isolating the desired amine of Formula I, by extraction with a suitable solvent preferably toluene or methylene chloride.

The (4*R*)-1-benzoyl-4-hydroxy-L-proline can be isolated from the aqueous solution by acidification and recycled, which further adds to the efficiency of the process.

The (αS)-α-(2-methylpropyl)-2- (1-piperidinyl)benzenemethanamine of Formula I, is further converted to repaglinide of Formula II, by condensing with 3-ethoxy-4-(ethoxycarbonyl)benzene acetic acid.

The present invention also relates to the novel diastereomeric salt of Formula IV

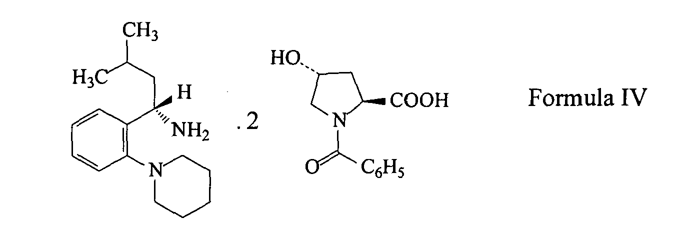

The invention is illustrated with the following examples, which are provided by way of illustration only and should not be construed to limit the scope of the invention.

### EXAMPLE 1

### PREPARATION OF SALT OF (αS)-α-(2-METHYLPROPYL)-2-(1-PIPERIDINYL)BENZENEMETHANAMINE WITH (4R)-1-BENZOYL-4-HYDROXY-L-PROLINE

α-(2-Methylpropyl)-2-(1-piperidinyl)benzenemethanamine (1g, 0.004 moles) was added to a stirred suspension of (4R)-1-benzoyl-4-hydroxy-L-proline (1.9 g, 0.008 moles) in acetone (20 ml) at 25-30°C. The reaction mixture was heated to 40-45°C to obtain a clear solution. As crystals separated out, refluxing was continued for 10 min, the suspension was cooled to 25-30°C and product was filtered and dried. The solid (yield: 1.45 g; enantiomeric purity 93.93% HPLC) was suspended in acetone (15 ml) and stirred at 50°C for 10 min. The mixture was filtered and title solid product obtained was dried under reduced pressure.
**Yield**: 1.2g (82.4%)
**Enantiomeric purity**: 97.54% by HPLC.

### EXAMPLE 2

### PREPARATION OF SALT OF (αS)-α-(2-METHYLPROPYL)-2-(1-PIPERIDINYL)BENZENEMETHANAMINE WITH (4R)-1-BENZOYL-4-HYDROXY-L-PROLINE

α-(2-Methylpropyl)-2-(1-piperidinyl)benzenemethanamine (5g, 0.0203 moles) was added to a stirred suspension of (4R)-1-benzoyl-4-hydroxy-L-proline (4.8 g, 0.0203 moles) in a mixture of acetone (10 ml) and ethyl acetate (90 ml) at 25-30°C. The reaction mixture was heated to reflux and was cooled slowly to 25-30°C and stirred for 40 min. Filtered the solid obtained and dried.
**Yield:** 5.5 g
**Enantiomeric purity**: 96.37 % by HPLC

The above obtained salt of (α*s*)-α-(2-methylpropyl)-2-(1-piperidinyl)benzene-methanamine with (4*r*)-1-benzoyl-4-hydroxy-1-proline (0.5 g) was crystallized from a mixture of methanol/ethylacetate (1:5; 12 ml).
**Yield**: 0.3 g
**Enantiomeric purity**: 100.00 % by HPLC.
[α]²⁰_{D} = -81.1 (C=1 in methanol)
**¹H NMR** (DMSO-D₆): δ PPM 7.57-7.23 (m, 14H), 4.71 (m, 1H). 4.48-4.09 (m, 4H), 2.05-1.93 (m, 4H), 1.63-1.4 (m, 9H), 0.91 (d, 2H), 0.83 (d, 2H)

### EXAMPLE 3

### PREPARATION OF (αS)-α-(2-METHYLPROPYL)-2-(1-PIPERIDI-NYL)BENZENEMETHANAMINE

α-(2-Methylpropyl)-2-(1-piperidinyl)benzenemethanamine (10 g, 0.04 moles) was added to a stirred suspension of (4*R*)-1-benzoyl-4-hydroxy-L-proline (9.6 g, 0.04 moles) in acetone (200 ml) and warmed to 40°C to form a clear solution. As solid crystallized out, the reaction mixture was heated to reflux for 30 min. The suspension was cooled to 25-30°C, stirred for 1 h, filtered and the product was dried. The solid (9 g) was further suspended in acetone (90 ml) and stirred at 50°C. It was filtered under suction, washed with acetone (20 ml) and dried. **Yield**: 7.9 g
**Enantiomeric purity**: 98.8 %

Salt of (α*S*)-α-(2-methylpropyl)-2-(1-piperidinyl)benzenemethanamine with (4*R*)-1-benzoyl-4-hydroxy-L-proline (6 g; enantiomeric purity 98.8 %) obtained above was suspended in DM water (90 ml) and aqueous sodium hydroxide solution (1.0 ml, 50% w/v) was added at 10-15°C. The reaction mixture was extracted with methylene chloride (3 x 25 ml) and the combined organic layer was washed with DM water and distilled under reduced pressure to furnish product.
**Yield**: 2 g
Enantiomeric purity: 98.27 % by HPLC

### EXAMPLE 4

### PREPARATION OF SALT OF (αS)-α-(2-METHYLPROPYL)-2-(1-PIPERIDINYL)BENZENEMETHANAMINE

α-(2-Methylpropyl)-2-(1-piperidinyl)benzenemethanamine (10 g, 0.04 moles) was added to a stirred suspension of (4R)-1-benzoyl-4-hydroxy-L-proline (19.1 g, 0.081 moles) in acetone (200 ml) and heated to reflux at 52-55°C for 10 h and cooled slowly to 25-30°C and stirred for 1 h. The obtained solid was filtered and dried.
**Yield**: 11 g
**Enantiomeric purity** 100.0 % by HPLC

The above obtained salt (11 g) was suspended in DM water (165 ml) and cooled to 10-15°C. Aq. Sodium hydroxide solution (50 % w/v; 3 ml) was added to the suspension. Thereafter, the reaction mass was extracted with methylene chloride (2 x 100 ml) and washed with water (33 ml). The methylene chloride layer was concentrated to obtain (α*s*)-α-(2-methylpropyl)-2-(1-piperidinyl)benzenemethanamine.
**Yield**: 3.7 g
**Enantiomeric purity**: 99.14 % by HPLC.

### EXAMPLE 5

### PREPARATION OF REPAGLINIDE

3-Ethoxy-4-(ethoxycarbonyl)benzene acetic acid (2.25 g, 0.0089 moles) was added to a solution of (αS)-α-(2-methylpropyl)-2-(1-piperidinyl)benzenemethanamine (2g, .008 moles) in toluene (20 ml). Then *N,N*-dicyclohexylcarbodiimide (1.95 g, 0.0094 moles) was added and the reaction mixture was stirred till completion of the reaction at 25-40°C. The solid was filtered under suction and toluene filtrate was distilled under reduced pressure. The product was crystallized from ethanol / water and dried to yield ethyl 2-ethoxy-4-[2-[[(1S)-3-methyl-1-[2-(1-piperidinyl)phenyl]butyl]amino]-2-oxoethyl]benzoate (3 g). 1*N* aqueous sodium hydroxide solution (8.0 ml) in ethanol (30 ml) was added to ethyl-2-ethoxy-4-[2-[[(1S)-3-methyl-1-[2-(1-piperidinyl)phenyl]butyl]amino]-2-oxoethyl]benzoate (3 g) at 60-65°C and stirred. After completion of the reaction the reaction mixture was cooled, pH was adjusted to 5 with 1*N* aqueous hydrochloric acid and product was filtered and dried at reduced pressure. The dried product was recrystallised from aqueous ethanol to give repaglinide.
**Yield**: 2.27 g

## Claims

1. An improved process for the preparation of (αS)-α-(2-methylpropyl)-2-(1-piperidinyl)benzenemethanamine of Formula I 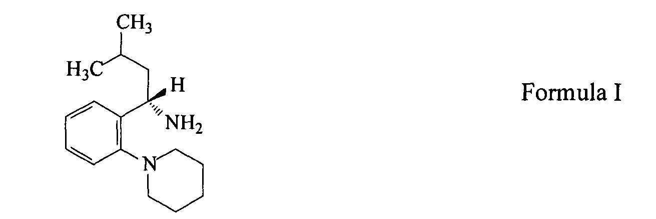 which comprises:
a) dissolving racemic amine of Formula III 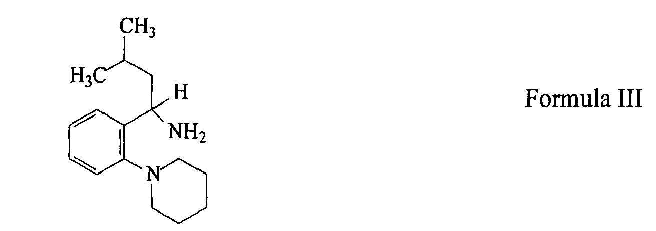 in a solvent or a mixture of solvents;
b) adding an (4*R*)-1-benzoyl-4-hydroxy-L-proline; and
c) crystallizing the diastereomeric salt and isolating the amine of Formula I.

2. The process according to claim 1, wherein the solvent is selected from aliphatic esters such as ethyl acetate, ketones such as acetone, alcohols such as methanol, and ethers such as t-butyl methyl ether.

3. The process according to claim 2, wherein the solvent is acetone.

4. The novel diastereomeric salt of Formula IV

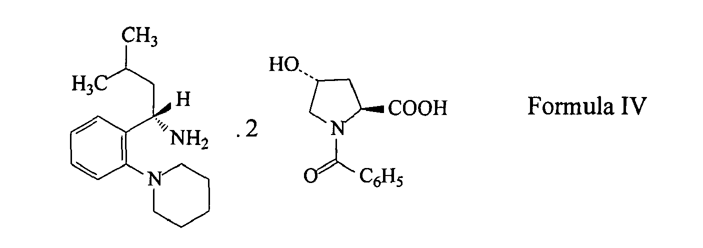

5. A process for the preparation of Repaglinide (Formula II) comprising the method steps according to any of claims 1-3.

6. A process for the preparation of Repaglinide (Formula II) comprising the utilization of the diastereomeric salt of Formula IV.
